(19) Europäisches Patentamt
European Patent Office
Office européen des brevets

(11) EP 1 537 177 B1

# (12) EUROPÄISCHE PATENTSCHRIFT

(45) Veröffentlichungstag und Bekanntmachung des Hinweises auf die Patenterteilung:
**16.08.2006 Patentblatt 2006/33**

(21) Anmeldenummer: **03794935.1**

(22) Anmeldetag: **25.08.2003**

(51) Int Cl.:
***C08L 39/02*** *(2006.01)* ***C08L 101/00*** *(2006.01)*
***C08L 57/00*** *(2006.01)* ***A61L 15/24*** *(2006.01)*
***A61L 15/60*** *(2006.01)*

(86) Internationale Anmeldenummer:
**PCT/EP2003/009406**

(87) Internationale Veröffentlichungsnummer:
**WO 2004/024816 (25.03.2004 Gazette 2004/13)**

## (54) WASSERABSORBIERENDES MITTEL UND VERFAHREN ZU SEINER HERSTELLUNG

WATER ABSORBING AGENT AND METHOD FOR THE PRODUCTION THEREOF

AGENT D'ABSORPTION D'EAU ET PROCEDE POUR LE PREPARER

(84) Benannte Vertragsstaaten:
**AT BE BG CH CY CZ DE DK EE ES FI FR GB GR HU IE IT LI LU MC NL PT RO SE SI SK TR**

(30) Priorität: **26.08.2002 DE 10239074**

(43) Veröffentlichungstag der Anmeldung:
**08.06.2005 Patentblatt 2005/23**

(73) Patentinhaber: **BASF Aktiengesellschaft**
**67056 Ludwigshafen (DE)**

(72) Erfinder:
- **DANIEL, Thomas**
  **67165 Waldsee (DE)**
- **RIEGEL, Ulrich**
  **60386 Frankfurt (DE)**
- **ELLIOTT, Mark**
  **67063 Ludwigshafen (DE)**
- **WEISMANTEL, Matthias**
  **63637 Jossgrund-Oberndorf (DE)**
- **WENDKER, Martin**
  **67549 Worms (DE)**

(74) Vertreter: **Reitstötter - Kinzebach**
**Ludwigsplatz 4**
**67059 Ludwigshafen (DE)**

(56) Entgegenhaltungen:
**EP-A- 0 761 241** **GB-A- 811 135**
**US-A- 4 987 182** **US-A- 6 121 409**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 537 177 B1

Printed by Jouve, 75001 PARIS (FR)

**Beschreibung**

Die vorliegende Erfindung betrifft ein wasserabsorbierendes Mittel sowie Verfahren zu seiner Herstellung

Wasserabsorbierende Polymerisate, die auch als Hydrogel-bildende Polymerisate oder Superabsorber (Superabsorbing Polymers, im Folgenden als SAP abgekürzt) bezeichnet werden, sind bekannt. Hierbei handelt es sich um Netzwerke flexibler hydrophiler Polymerisate, die sowohl ionischer als auch nichtionischer Natur sein können. Diese sind in der Lage, wässrige Flüssigkeiten unter Bildung eines Hydrogels zu absorbieren und damit zu binden. Einen umfassenden Überblick über SAP, ihre Anwendung und ihre Herstellung gibt F.L. Buchholz und A.T. Graham (Herausgeber) in "Modern Superabsorbent Polymer Technology", Wiley-VCH, New York, 1998.

SAP finden insbesondere in Hygieneartikeln wie Windeln, Inkontinenzeinlagen und -hosen, Damenbinden und dergleichen zur Absorption von Körperflüssigkeiten Verwendung. In den letzten Jahren besteht eine zunehmende Tendez, die Hygieneartikel aus ästhetischen Gründen und unter Umweltaspekten immer dünner und kleiner zu gestalten. Hierzu ist es erforderlich, dass die SAPs über eine gute Flüssigkeitsverteilung und -weiterleitung sowie über eine hohe Quellfähigkeit verfügen. Problematisch ist hierbei vielfach, dass der Superabsorber an der Eintrittsstelle der Flüssigkeit stark quillt und eine Sperrschicht für nachfolgende Flüssigkeitsmengen bildet. Hierdurch wird eine Weiterleitung und Verteilung der Flüssigkeit im Absorptionskern verhindert. Diese Eigenart des Superabsorbers wird auch als "Gelblocking-Effekt" bezeichnet. Nachfolgende Flüssigkeitsmengen werden dann nicht mehr vom Absorptionskern aufgenommen, und es kommt zur unkontrollierten Verteilung der Flüssigkeit auf der Windeloberfläche und im Extremfall zum Austritt der Flüssigkeit. In diesem Zusammenhang ist es auch wichtig, dass die SAP im gequollenem Zustand über eine hohe Gelfestigkeit verfügen. Gele mit nur einer geringen Gelfestigkeit werden unter Druck, z. B. Körperdruck, deformiert und verstopfen die Poren des Absorptionskerns.

Die Gelfestigkeit und damit die Permeabilität eines SAPs lässt sich zwar durch Erhöhung der Vernetzungsdichte verbessern, jedoch verringert dies das Endabsorptionsvermögen des SAPs. Um eine hohe Permeabilität zu erreichen, müssen die Poren im SAP einen großen Durchmesser aufweisen und muss somit der Anteil an SAP-Teilchen geringer Korngröße in wirtschaftlich und technisch nachteiliger Weise verringert werden.

Ein weiteres Problem wasserquellbarer Polymere ist ihr extrahierbarer Anteil. Wasserquellbare Polymere enthalten stets herstellungsbedingt einen gewissen Anteil an extrahierbaren Bestandteilen wie Natriumpolyacrylat. Zum anderen kann das polymere Netzwerk beim Quellen reißen, so dass unter Anwendungsbedingungen sich der Anteil an Extrahierbaren weiter erhöht. Dies gilt insbesondere für stark gequollene Gele. Beim Kontakt der wasserquellbaren Polymerisate mit Körperflüssigkeiten werden die extrahierbaren Anteile ausgewaschen. Sie verdicken die Flüssigkeit, wandern an die Oberfläche und verhindern damit ein Eindringen weiterer Flüssigkeit, d. h. je höher der Anteil an Extrahierbaren ist um so geringer ist die Flüssigkeitsweiterleitung.

Die EP-A 0 761 241 beschreibt absorbierende Mittel, die ein absorbierendes Harz und ein wasserunlösliches anorganisches Pulver und/oder ein Polyamin mit einem mittleren Molekulargewicht von wenigstens 5000 enthalten. Das wasserunlösliche anorganische Pulver ist vorzugsweise Siliciumdioxid und das Polyamin ist vorzugsweise Polyethylenimin.

Zur Verbesserung der Quellfähigkeit und der Flüssigkeitsweiterleitung sowie zur Erhöhung der Nassfestigkeit schlägt die US 6,099,950 die Mitverwendung von Polymeren wie Polyaminen und Polyiminen, wobei das Polymer mit wenigstens einem Bestandteil des Urins zu reagieren vermag, in wasserabsorbierenden Mitteln vor. Zu den geeigneten Polyaminen zählt beispielsweise Polyvinylamin und Polyallylamin.

Zur Verbesserung der Gelpermeabiltiät bei gleichbleibender Quellfähigkeit empfiehlt die ältere deutsche Patentanmeldung 101 02 429.0 wasserabsorbierende Mittel, die Teilchen eines wasserabsorbierenden Polymerisats enthalten, deren Oberfläche mit einem wasserunlöslichen Metallphosphat assoziiert ist.

Das Eigenschaftsprofil der beschriebenen wasserabsorbierenden Mittel ist jedoch nicht in allen Punkten zufriedenstellend. Es besteht ein Bedürfnis nach wasserabsorbierbaren Mitteln, die sowohl eine sehr hohe Flüssigkeitsweiterleitung oder Permeabilität (SFC) aufweisen, d. h. Flüssigkeit sehr gut durch die gequollene Gelschicht hindurchlassen, als auch über ein sehr hohes Absorptionsvermögen verfügen. Ferner sollten sich die absorbierenden Mittel durch eine hohe Nassfestigkeit auszeichnen.

Der vorliegenden Erfindung liegt daher die Aufgabe zugrunde, wasserabsorbierende Mittel bereitzustellen, die sich durch verbesserte anwendungstechnische Eigenschaften, insbesondere durch eine gesteigerte Flüssigkeitsdurchlässigkeit (Permeabilität), hohe Absorptionskapazitäten sowohl unter Druck als auch unter freien Quellbedingungen und eine hohe Nassfestigkeit auszeichnen.

Erfindungsgemäß wird die Aufgabe gelöst durch ein wasserabsorbierendes Mittel, das umfasst

(a) Teilchen eines wasserabsorbierenden Polymerisats, und
(b) ein stickstoffhaltiges Polymer, das 5 bis 17 mol/kg, vorzugsweise 5,5 bis 15 mol/kg, insbesondere 6,0 bis 12 mol/kg und ganz besonders bevorzugt 6,5 bis 10 mol/kg, bezogen auf das Gesamtgewicht des stickstoffhaltigen Polymers, protonierbare Stickstoffatome enthält.

Die vorliegende Erfindung betrifft außerdem ein Verfahren zur Herstellung des erfindungsgemäßen wasserabsorbierenden Mittels.

Das erfindungsgemäße wasserabsorbierende Mittel weist üblicherweise eine oder mehrere, insbesondere alle, der folgenden Eigenschaften auf:

- eine Korngrößenverteilung, worin mehr als 98 Gew.-% der Teilchen eine Korngröße von 100 bis 850 μm, vorzugsweise 100 bis 600 μm und insbesondere 100 bis 500 μm aufweisen;
- Saline Flow Conductivity von wenigstens 30 x $10^{-7}$ $cm^3 \cdot s/g$, vorzugsweise von wenigstens 60 x $10^{-7}$ $cm^3 \cdot s/g$ und insbesondere von wenigstens 100 x $10^{-7}$ $cm^3 \cdot s/g$;
- Ball Burst Strength (30 min) [BBS (30 min)] von wenigstens 50 gf, vorzugsweise wenigstens 90 gf und insbesondere wenigstens 110 gf;
- Ball Burst Strength (16 h) [BBS (16 h)] von wenigstens 50 gf, vorzugsweise wenigstens 75 gf und insbesondere wenigstens 100 gf;
- einen Quotienten [BBS (30 min) - BBS (16 h)]/BBS (30 min) von weniger als 0,8, vorzugsweise weniger als 0,5 und insbesondere weniger als 0,4;
- CRC von 20 bis 33 g/g;
- eine AUL (0,7 psi) von 17 bis 27 g/g.

Es hat sich überraschenderweise gezeigt, dass die Eigenschaften des wasserabsorbierenden Mittels, wie insbesondere die Flüssigkeitsweiterleitung (SFC), Quellfähigkeit (CRC), Absorption unter Druck (AUL 0,7 psi), stark vom Anteil der protonierbaren Stickstoffatome im stickstoffhaltigen Polymer (im Folgenden auch: "Ladungsdichte") abhängen. So erzielt man mit stickstoffhaltigen Polymeren mit einer Ladungsdichte im angegebenen Bereich eine deutlich verbesserte Flüssigkeitsweiterleitung (SFC), eine verbesserte Quellfähigkeit (CRC), verbesserte Absorption unter Druck (AUL 0,7 psi) bei vergleichbarer Nassfestigkeit (BBS) als mit stickstoffhaltigen Polymeren mit einer Ladungsdichte außerhalb des angegebenen Bereichs.

Unter "protonierbaren Stickstoffatomen" werden Stickstoffatome in funktionellen Gruppen angesehen, die in wässrigem Medium (prinzipiell) protonierbar sind, unabhängig davon, ob die Gruppen bei einem bestimmten pH-Wert tatsächlich protoniert vorliegen. Vorzugsweise handelt es sich um primäre Aminogruppen.

Zu den geeigneten stickstoffhaltigen Polymeren zählen die Produkte der kontrollierten Hydrolyse von Homo- und Copolymeren von N-Vinylcarbonsäureamiden und/oder N-Vinylcarbonsäureimiden. Bei der kontrollierten Hydrolyse wird von einem Teil der einpolymerisierten N-Vinylcarbonsäureamid- oder N-Vinylcarbonsäureimid-Einheiten die Acylgruppe(n) durch Einwirkung von Säuren, Basen oder Enzymen unter Bildung von Vinylamineinheiten abgespalten.

Als N-Vinylcarbonsäureamide kommen grundsätzlich offenkettige und cyclische N-Vinylcarbonsäureamide in Betracht. Bevorzugte N-Vinylcarbonsäureamide sind offenkettige N-Vinylcarbonsäureamide, insbesondere solche offenkettige N-Vinylcarbonsäureamide, deren Hydrolyse ein primäres Amin liefert. Beispiele für besonders geeignete N-Vinylcarbonsäureamide sind N-Vinylformamid, N-Vinylacetamid und N-Vinylpropionamid, insbesondere N-Vinylformamid. Beispiele für geeignete N-Vinylimide sind N-Vinylsuccinimid und N-Vinylphthalimid. Die genannten Monomeren können entweder allein oder in Mischung untereinander polymerisiert werden.

In einer bevorzugten Ausführungsform handelt es sich bei dem stickstoffhaltigen Polymer um ein Homopolymerisat des N-Vinylformamids mit einem Hydrolysegrad von 30 bis 80 mol-%, vorzugsweise 40 bis 60 mol.-%. Die Herstellung derartiger teilweise hydrolysierter Polyvinylamide (umgangssprachlich auch "teilhydrolysierte Polyvinylamine" genannt) ist beispielsweise in der DE 31 28 478 beschrieben, auf die hiermit im vollem Umfang Bezug genommen wird. Lösungen vollständig oder teilweise hydrolysierter Polyvinylformamide sind im Handel erhältlich und werden beispielsweise von der BASF Aktiengesellschaft unter den Handelsnamen Basocoll®, Luredur® sowie Catiofast® vertrieben.

Die Hydrolyse lässt sich besonders vorteilhaft im alkalischen Medium durchführen, beispielsweise in einem pH-Bereich von 9 bis 14. Dieser pH-Wert wird vorzugsweise durch Zugabe von wässrigem Alkalihydroxid wie Natronlauge oder Kalilauge eingestellt. Es ist jedoch auch möglich, Ammoniak, Amine und Erdalkalihydroxide wie Calciumhydroxid zu verwenden. Die Hydrolyse lässt sich ebenfalls im saurem Medium durchführen, beispielsweise in einem pH-Bereich von 0 bis 3. Geeignete Säuren sind Carbonsäuren, wie Ameisensäure, Essigsäure, Propionsäure, Sulfonsäuren wie Benzolsulfonsäure oder Toluolsulfonsäure oder anorganische Säuren wie Salzsäure, Schwefelsäure, Phosphorsäure oder Bromwasserstoffsäure.

Über die Konzentration an Säure oder Base lässt sich der Hydrolysegrad des Polyvinylamins einstellen. Üblicherweise setzt man pro Mol zu hydrolysierendes Acylgruppenäquivalent im stickstoffhaltigen Polymer die Base in äquimolarer Menge ein, d. h. für einen Hydrolysegrad von 30 bis 80 Mol % verwendet man üblicherweise pro Acylgruppenäquivalent im stickstoffhaltigen Polymer 0,3 bis 0,8 Äquivalente einer Base, für einen Hydrolysegrad von 40 bis 60 Mol.-% verwendet man üblicherweise pro Acylgruppenäquivalent im stickstoffhaltigen Polymer 0,4 bis 0,6 Äquivalente einer Base.

Die Hydrolyse kann in verschiedenen Solventien wie Wasser, Alkoholen, Ammoniak und Aminen oder Mischungen, z. B. aus Wasser und Alkoholen oder wässrigen Lösungen von Ammoniak und/oder Aminen erfolgen. Vorzugsweise erfolgt die Hydrolyse in Wasser oder in Alkoholen wie Methanol, Ethanol, Isopropanol, n-Butanol. Die Nebenprodukte der Hydrolyse werden während oder nach der Hydrolyse aus dem System entfernt. Die Reaktionstemperatur liegt üblicherweise in einem Temperaturbereich von 40 bis 180°C. Gegebenenfalls entfernt man das Lösungsmittel vollständig oder engt auf den gewünschten Gehalt ein.

Alternativ kann man als stickstoffhaltiges Polymer Copolymere verwenden, die einpolymerisierte Einheiten von Monomeren mit protonierbare Stickstoffatome umfassenden Seitengruppen und von Monomeren ohne protonierbare Stickstoffatome in einem geeigneten Verhältnis enthalten. Die Ladungsdichte LD errechnet sich nach der folgenden Gleichung:

$$LD = \frac{x_N}{x_N M_N + x_O M_O}$$

worin $x_N$ für den molaren Anteil des protonierbare Stickstoffatome umfassenden Monomers im Copolymer, $x_o$ für den molaren Anteil des Monomers ohne protonierbare Stickstoffatome im Copolymer, $M_N$ für das Molekulargewicht des protonierbare Stickstoffatome umfassenden Monomers und $M_o$ für das Molekulargewicht des Monomers ohne protonierbare Stickstoffatome steht. Der Begriff "molarer Anteil" bezieht sich hierbei auf die Zusammensetzung einer für das gegebene Polymer repräsentativen Polymerkette.

Geeignete Monomere mit protonierbare Stickstoffatome umfassenden Seitengruppen sind z.B. Allylamin, Di($C_1$-$C_4$-alkyl)aminoethylacrylat, N-Di($C_1$-$C_4$-alkyl)aminoethyl-acrylamid, N-Di($C_1$-$C_4$-alkyl)aminopropylacrylamid und dergleichen.

Anstelle der Monomeren mit protonierbare Stickstoffatome umfassenden Seitengruppen kann man auch Vorläufer-Monomere einpolymerisieren, aus denen die protonierbaren Stickstoffatome durch geeignete Nachbehandlung freigesetzt werden. Die Seitengruppen der Vorläufer-Monomere können z. B. geschützte Aminogruppen enthalten, aus denen nach der Polymerisation die Schutzgruppe abgespalten wird. Geeignete Vorläufer-Monomere sind die weiter oben genannten N-Vinylcarbonsäureamide und/oder N-Vinylcarbonsäureimide, aus denen durch vollständige oder teilweise Hydrolyse Vinylamineinheiten entstehen. Die Menge an Monomeren ohne protonierbare Stickstoffatome und/oder der Hydrolysegrad der Einheiten der Vorläufer-Monomere wird so eingestellt, dass die angestrebte Ladungsdichte erhalten wird. Bevorzugt sind Copolymere, worin sämtliche Einheiten der Vorläufer-Monomere, wie N-Vinylformamid, hydrolysiert sind.

Geeignete Monomere ohne protonierbare Stickstoffatome sind ethylenisch ungesättigte $C_3$- bis $C_6$-Carbonsäuren, beispielsweise Acrylsäure, Methacrylsäure, Maleinsäure, Crotonsäure, Itakonsäure und Vinylessigsäure. Weiterhin sind als Comonomere geeignet: Vinylacetat, Acrylnitril, Methacrylnitril.

Als stickstoffhaltiges Polymer ist z. B. das Hydrolyseprodukt eines Copolymers von Vinylformamid und Acrylsäure geeignet. Der Vinylformamidanteil beträgt beispielsweise 40 bis 80 mol-% und der Acrylsäureanteil 60 bis 20 mol-%.

Das stickstoffhaltige Polymer weist vorzugsweise ein gewichtsmittleres Molekulargewicht von 10000 bis 500000 Dalton, vorzugsweise 50000 bis 450000 Dalton, insbesondere 100000 bis 420000 Dalton auf.

Das stickstoffhaltige Polymer wird üblicherweise in einer Menge von 0,001 bis 5 Gew.-%, vorzugsweise 0,01 bis 2 Gew.-%, insbesondere bis 1,5 Gew.-% und ganz besonders bevorzugt bis 1,0 Gew.-%, bezogen auf das Gewicht des wasserabsorbierenden Polymerisats, verwendet.

In einer Ausführungsform enthält das erfindungsgemäße wasserabsorbierende Mittel außerdem wenigstens ein feinteiliges, wasserunlösliches Salz, das unter anorganischen und organischen Salzen und Gemischen davon ausgewählt ist. Unter einem wasserunlöslichen Salz versteht man im Rahmen der vorliegenden Erfindung ein Salz, das bei einem pH-Wert von 7 eine Wasserlöslichkeit von weniger als 5 g/l, vorzugsweise von weniger als 3 g/l, insbesondere von weniger als 2 g/l und ganz besonders bevorzugt von weniger als 1 g/l (bei 25 °C und 1 bar) aufweist. Die Mitverwendung des wasserunlöslichen Salzes kann die durch das stickstoffhaltige Polymer verursachte Klebrigkeit des wasserabsorbierenden Mittels während des Auftragens herabsetzen.

Geeignete Kationen in dem wasserunlöslichen Salz sind beispielsweise $Ca^{2+}$, $Mg^{2+}$, $Al^{3+}$, $Sc^{3+}$, $Y^{3+}$, $Ln^{3+}$ (wobei Ln für Lanthanoide steht), $Ti^{4+}$, $Zr^{4+}$, $Li^{+}$ oder $Zn^{2+}$. Geeignete anorganische anionische Gegenionen sind beispielsweise Carbonat, Sulfat, Hydrogencarbonat, Orthophosphat, Silicat,Oxid oder Hydroxid. Geeignete anionische, organische Gegenionen sind beispielsweise Oxalat und Tartrat. Zweckmäßigerweise wählt man das Gegenion so aus, dass es mit dem stickstoffhaltigen Polymer keine wasserunlöslichen Komplexe bildet. Sofern ein Salz in verschiedenen Modifikationen vorkommt, sind alle Modifikationen des Salzes eingeschlossen. Vorzugsweise sind die wasserunlöslichen anorganischen Salze ausgewählt unter Calciumsulfat, Calciumcarbonat, Calciumphosphat, Calciumsilicat, Calciumfluorid, Apatit, Magnesiumphosphat, Magnesiumhydroxid, Magnesiumoxid, Magnesiumcarbonat, Dolomit, Lithiumcarbonat, Lithiumphosphat, Zinkoxid, Zinkphosphat, Oxiden, Hydroxiden, Carbonaten und Phosphaten der Lanthanoiden, Natriumlanthanoidsulfat, Scandiumsulfat, Yttriumsulfat, Lanthansulfat, Scandiumhydroxid, Scandiumoxid, Aluminiumoxid, hydratisiertem Aluminiumoxid und Gemischen davon. Unter dem Begriff Apatit versteht man die Fluor-, Hydroxyl-, Chlor-, Carbonat- und Carbonat-Fluorapatite. Vorzugsweise sind die organischen wasserunlöslichen Salze ausgewählt unter Calciumoxalat, Scandiumoxalat, den Oxalaten der Lanthanoiden und Gemischen davon. Insbesondere eignen sich Calcium- und Magnesiumsalze wie Calciumcarbonat, Calciumphosphat, Magnesiumcarbonat, Calciumoxid, Magnesiumoxid, Calciumsulfat und Gemische davon.

Das feinteilige wasserunlösliche Salz weist üblicherweise eine mittlere Teilchengröße von weniger als 500 µm, vorzugsweise von weniger als 200 µm, insbesondere von weniger als 100 µm, besonders bevorzugt weniger als 50 µm und ganz besonders bevorzugt von weniger als 20 µm und am stärksten bevorzugt im Bereich von 1 bis 10 µm auf.

Falls verwendet, wird das feinteilige, wasserunlösliche Salz in einer Menge von 0,001 bis 20 Gew.-%, vorzugsweise weniger als 10 Gew.-%, insbesondere 0,001 bis 5 Gew.-%, ganz besonders bevorzugt 0,001 bis 2 Gew.-%, und am stärksten bevorzugt zwischen 0,001 und 1 Gew.-%, bezogen auf das Gewicht des wasserabsorbierenden Polymerisats, eingesetzt.

Um ein Stauben des feinteiligen wasserunlöslichen Salzes und/oder das Entmischen des feinteiligen wasserunlöslichen Salzes und des wasserabsorbierenden Polymerisats zu verhindern, kann es sich empfehlen, das feinteilige wasserunlösliche Salz und/oder das wasserabsorbierenden Polymerisat mit einem Staubbindemittel zu benetzen. Als Staubbindemittel eignen sich niedermolekulare Verbindungen, die weniger flüchtig sind als Wasser und vorzugsweise physiologisch unbedenklich sind. Geeignete Staubbindemittel sind beispielsweise Polyole, vorzugsweise $C_1$-$C_{10}$-Polyhydroxyverbindungen wie 1,2-Propandiol, 1,3-Propandiol, 1,4-Butandiol, Glycerin, Sorbit. Ein bevorzugtes Staubbindemittel ist 1,2-Propandiol.

Das Staubbindemittel wird in einer Menge bis zu 6 Gew.-%, vorzugsweise bis 3 Gew.-%, insbesondere bis 1 Gew.-%, besonders bevorzugt bis 0,1 Gew.-%, bezogen auf das Gewicht des wasserabsorbierenden Polymerisats, verwendet.

Als wasserabsorbierende Polymerisate eignen sich alle im Stand der Technik bekannten wasserabsorbierenden Polymerisate. Geeignete wasserabsorbierende Polymerisate sind insbesondere Polymerisate hydrophiler Monomere, Pfropf(co)polymere eines oder mehrerer hydrophiler Monomere auf einer geeigneten Pfropfgrundlage, vernetzte Cellulose- oder Stärkeether, vernetzte Carboxymethylcellulose, teilweise vernetzte Polyether oder in wässrigen Flüssigkeiten quellbare Naturprodukte, wie Guarderivate, Alginate oder Carrageenane.

Geeignete Pfropfgrundlagen können natürlichen oder synthetischen Ursprungs sein. Hierzu zählen Stärken, d. h. native Stärken aus der Gruppe der Maisstärke, Kartoffelstärke, Weizenstärke, Reisstärke, Tapiokastärke, Sorghumstärke, Maniokstärke, Erbsenstärke 5 oder deren Mischungen, modifizierte Stärken, Stärkeabbauprodukte, z. B. oxidativ, enzymatisch oder hydrolytisch abgebaute Stärken, Dextrine, z. B. Röstdextrine sowie niedere Oligo- und Polysaccharide, z. B. Cyclodextrine mit 4 bis 8 Ringgliedern. Als Oligo-und Polysaccharide kommen weiterhin Cellulose, Stärke- und Cellulosederivate in Betracht. Ferner eignen sich Polyvinylalkohole, Polyamine, Polyamide, hydrophile Polyester oder Polyalkylenoxide, insbesondere Polyethylenoxid und Polypropylenoxid. Geeignete Polyalkylenoxide weisen die allgemeine Formel I auf,

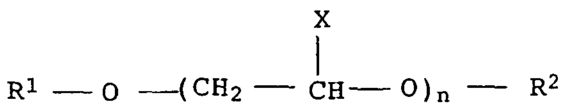

(I)

worin

- $R^1$, $R^2$ unabhängig voneinander für Wasserstoff; $C_1$-$C_4$-Alkyl; $C_2$-$C_6$-Alkenyl; Aryl, insbesondere Phenyl; oder (Meth) acryloyl stehen;
- X für Wasserstoff oder Methyl und
- n für eine ganze Zahl von 1 bis 1000, insbesondere 10 bis 400 steht.

Vorzugsweise verwendet man als wasserabsorbierende Polymerisate Polymere monoethylenisch ungesättigter Säuren. Diese liegen vorzugsweise zumindest teilweise in Form ihrer Salze, insbesondere der Alkalimetallsalze, wie Natrium- oder Kaliumsalze, oder als Ammoniumsalze vor. Derartige Polymerisate quellen beim Kontakt mit wässrigen Flüssigkeiten besonders gut zu Gelen auf.

Besonders bevorzugt sind vernetzte wasserabsorbierende Polymerisate monoethylenisch ungesättigter $C_3$-$C_6$-Carbonsäuren und/oder deren Alkali- oder Ammoniumsalze. Insbesondere werden vernetzte Polyacrylsäuren bevorzugt, deren Säurengruppen zu 25 bis 100 % als Alkali- oder Ammoniumsalze vorliegen.

Derartige Polymere erhält man z. B. durch Polymerisation monoethylenisch ungesättigter Säuren oder deren Salzen in Gegenwart von Vernetzern. Allerdings kann man auch ohne Vernetzer polymerisieren und nachträglich vernetzen.

Das wasserabsorbierende Polymerisat ist vorzugsweise aufgebaut aus

- 49,9 bis 99,9 Gew.-% wenigstens eines unter monoethylenisch ungesättigten Säuren und deren Salzen ausgewählten Monomers A,

- 0 bis 50 Gew.-%, vorzugsweise 0 bis 20 Gew.-% wenigstens eines von dem Monomeren A verschiedenen, nicht vernetzend wirkenden monoethylenisch ungesättigten Monomers B und

- 0,001 bis 20 Gew.-%, vorzugsweise 0,01 bis 14 Gew.-% wenigstens eines vernetzend wirkenden Monomers C.

Zu den Monomeren A zählen monoethylenisch ungesättigte Mono- und Dicarbonsäuren mit 3 bis 25 vorzugsweise 3 bis 6 C-Atomen, die auch in Form ihrer Salze oder Anhydride eingesetzt werden können. Beispiele hierfür sind Acrylsäure, Methacrylsäure, Ethacrylsäure, $\alpha$-Chloracrylsäure, Crotonsäure, Maleinsäure, Maleinsäureanhydrid, Itaconsäure, Citraconsäure, Mesaconsäure, Glutaconsäure, Aconitsäure und Fumarsäure. Zu den Monomeren A zählen weiterhin die Halbester von monoethylenisch ungesättigten Dicarbonsäuren mit 4 bis 10 vorzugsweise 4 bis 6 C-Atomen, z. B. von Maleinsäure wie Maleinsäuremonomethylester. Zu den Monomeren A zählen auch monoethylenisch ungesättigte Sulfonsäuren und Phosphonsäuren, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloyloxypropylsulfonsäure, 2-Hydroxy-3-methacryloyloxypropylsulfonsäure, Styrolsulfonsäure, 2-Acrylamido-2-methylpropansulfonsäure, Vinylphosphonsäure und Allylphosphonsäure und die Salze, insbesondere die Natrium-, Kalium- und Ammoniumsalze dieser Säuren. Die Monomere A können als solche oder als Mischungen untereinander eingesetzt werden. Die angegebenen Gewichtsanteile beziehen sich sämtlich auf die Säureform.

Bevorzugte Monomere A sind Acrylsäure, Methacrylsäure, Vinylsulfonsäure, 2-Acrylamido-2-methylpropansulfonsäure oder Mischungen dieser Säuren. Bevorzugte Monomere A sind Acrylsäure und Mischungen der Acrylsäure mit anderen Monomeren A, z. B. Mischungen aus Acrylsäure und Methacrylsäure, aus Acrylsäure und Acrylamidopropansulfonsäure oder aus Acrylsäure und Vinylsulfonsäure. Besonders bevorzugt umfassen die Monomere A als Hauptbestandteil Acrylsäure.

Zur Optimierung von Eigenschaften der erfindungsgemäßen Polymerisate kann es sinnvoll sein, zusätzliche monoethylenisch ungesättigte Monomere B einzusetzen, die von den Monomeren A verschieden sind, d. h. die keine Säuregruppen tragen, aber mit den Monomeren A copolymerisierbar sind und nicht vernetzend wirken. Hierzu gehören beispielsweise monoethylenisch ungesättigte Nitrile wie Acrylnitril, Methacrylnitril, die Amide der vorgenannten monoethylenisch ungesättigten Carbonsäuren, z. B. Acrylamid, Methacrylamid, N-Vinylamide wie N-Vinylformamid, N-Vinylacetamid, N-Methyl-vinylacetamid, N-Vinylpyrrolidon und N-Vinylcaprolactam. Zu den Monomeren zählen außerdem Vinylester gesättigter $C_1$-$C_4$-Carbonsäuren wie Vinylformiat, Vinylacetat und Vinylpropionat, Alkylvinylether mit mindestens 2 C-Atomen in der Alkylgruppe, z. B. Ethylvinylether oder Butylvinylether, Ester monoethylenisch ungesättigter $C_3$-$C_6$-Carbonsäuren, z. B. Ester aus einwertigen $C_1$-$C_{18}$-Alkoholen und Acrylsäure, Methacrylsäure oder Maleinsäure, Acrylsäure- und Methacrylsäureester von alkoxylierten einwertigen, gesättigten Alkoholen, z. B. von Alkoholen mit 10 bis 25 C-Atomen, die mit 2 bis 200 Mol Ethylenoxid und/oder Propylenoxid pro Mol Alkohol umgesetzt worden sind, sowie Monoacrylsäureester und Monomethacrylsäureester von Polyethylenglykol oder Polypropylenglykol, wobei die Molmassen ($M_n$) der Polyalkylenglykole beispielsweise bis zu 2000 betragen können. Weiterhin geeignete Monomere sind Styrol und alkylsubstituierte Styrole wie Ethylstyrol oder tert-Butylstyrol.

Als vernetzend wirkende Monomere C kommen solche Verbindungen in Betracht, die mindestens zwei ethylenisch ungesättigte Doppelbindungen im Molekül aufweisen. Beispiele für Verbindungen dieses Typs sind N,N'-Methylenbisacrylamid, Polyethylenglykoldiacrylate und Polyethylenglykoldimethacrylate, die sich jeweils von Polyethylenglykolen eines Molekulargewichts von 106 bis 8500, vorzugsweise 400 bis 2000, ableiten, Trimethylolpropantriacrylat, Trimethylolpropantrimethacrylat, Ethylenglykoldiacrylat, Ethylenglykoldimethacrylat, Propylenglykoldiacrylat, Propylenglykoldimethacrylat, Butandioldiacrylat, Butandioldimethacrylat, Hexandioldiacrylat, Hexandioldimethacrylat, Diethylenglykoldiacrylat, Diethylenglykoldimethacrylat, Triethylenglykoldiacrylat, Triethylenglykoldimethacrylat, Dipropylenglykoldiacrylat, Dipropylenglykoldimethacrylat, Tripropylenglykoldiacrylat, Tripropylenglykoldimethacrylat, Allylmethacrylat, Diacrylate und Dimethacrylate von Blockcopolymerisaten aus Ethylenoxid und Propylenoxid, zwei-, drei-, vier- oder fünffach mit Acrylsäure oder Methacrylsäure veresterte mehrwertige Alkohole, wie Glycerin, Trimethylolpropan, Pentaerythrit oder Dipentaerythrit, Ester monoethylenisch ungesättigter Carbonsäuren mit ethylenisch ungesättigten Alkoholen wie Allylalkohol, Cyclohexenol und Dicyclopentenylalkohol, z.B. Allylacrylat und Allylmethacrylat, weiterhin Triallylamin, Dialkyldiallylammoniumhalogenide wie Dimethyldiallylammoniumchlorid und Diethyldiallylammoniumchlorid, Tetraallylethylendiamin, Divinylbenzol, Diallylphthalat, Polyethylenglykoldivinylether von Polyethylenglykolen eines Molekulargewichtes von 106 bis 4000, Trimethylolpropandiallylether, Butandioldivinylether, Pentaerythrittriallylether, Umsetzungsprodukte von 1 Mol Ethylenglykoldiglycidylether oder Polyethylenglykoldiglycidylether mit 2 Mol Pentaerythritoltriallylether oder Allylalkohol, und Divinylethylenharnstoff.

Davon sind wasserlösliche Monomere bevorzugt, d. h. Verbindungen, deren Wasserlöslichkeit bei 20°C wenigstens 50 g/l beträgt. Hierzu zählen z. B. Polyethylenglykoldiacrylate und Polyethylenglykoldimethacrylate, Vinylether von Additionsprodukten von 2 bis 400 Mol Ethylenoxid an 1 Mol eines Diols oder Polyols, Ethylenglykoldiacrylat, Ethylenglykoldimethacrylat oder Triacrylate und Trimethacrylate von Additionsprodukten von 6 bis 20 Mol Ethylenoxid an 1 Mol Glycerin, Pentaerythrittriallylether und Divinylharnstoff.

Als Monomere C kommen weiterhin solche in Betracht, die wenigstens eine ethylenisch ungesättigte Doppelbindung sowie wenigstens eine weitere funktionelle Gruppe aufweisen, die hinsichtlich ihrer Reaktivität gegenüber Carboxylgruppen komplementär ist. Zu funktionellen Gruppen mit komplementärer Reaktivität gegenüber Carboxylgruppen zählen beispielsweise Hydroxyl-, Amino-, Epoxy- und Aziridinogruppen. Verwendung finden z. B. die Hydroxyalkylester der vorstehend erwähnten monoethylenisch ungesättigten Carbonsäuren, wie 2-Hydroxyethylacrylat, 3-Hydroxypropylacrylat, 4-Hydroxybutylacrylat, 2-Hydroxyethyl(meth)acrylat, 3-Hydroxypropyl(meth)acrylat und 4-Hydroxybutyl (meth)acrylat, Allylpiperidiniumbromid, N-Vinylimidazole, wie N-Vinylimidazol, 1-Vinyl-2-methylimidazol und N-Vinylimidazoline, wie N-Vinylimidazolin, 1-Vinyl-2-methylimidazolin, 1-Vinyl-2-ethylimidazolin oder 1-Vinyl-2-propylimidazolin, die in Form der freien Basen, in quaternisierter Form oder als Salz bei der Polymerisation verwendet werden. Außerdem eignen sich Dimethylaminoethylacrylat, Dimethylaminoethylmethacrylat, Diethylaminoethylacrylat oder Diethylaminoethylmethacrylat. Diese basischen Ester werden vorzugsweise in quarternisierter Form oder als Salz eingesetzt. Weiterhin eignet sich auch Glycid(meth)acrylat.

Als vernetzende Monomere C können ferner Verbindungen fungieren, die wenigstens zwei funktionelle Gruppen aufweisen, die hinsichtlich ihrer Reaktivität gegenüber der Carboxylgruppe des Polymers komplementär sind. Geeignete funktionelle Gruppen sind die bereits vorstehend genannten funktionellen Gruppen, wie Hydroxyl-, Amino-, Epoxy und Aziridingruppen sowie Isocyanat-, Ester- und Amidogruppen. Zu den geeigneten Vernetzern dieses Typs zählen beispielsweise Aminoalkohole, wie Ethanolamin oder Triethanolamin, Di- und Polyole, wie 1,3-Butandiol, 1,4-Butandiol, Ethylenglykol, Diethylenglykol, Triethylenglykol, Tetraethylenglykol, Polyethylenglykol, Glycerin, Polyglycerin, Propylenglykol, Polypropylenglykol, Trimethylolpropan, Pentaerythrit, Polyvinylalkohol, Sorbit, Stärke, Blockcopolymerisate aus Ethylenoxid und Propylenoxid, Polyamine wie Ethylendiamin, Diethylentriamin, Triethylentetramin, Tetraethylenpentamin, Pentaethylenhexamin und Polyethylenimine sowie Polyamine mit Molmassen von jeweils bis zu 4000000, Ester wie Sorbitanfettsäureester, ethoxylierte Sorbitanfettsäureester, Polyglycidylether wie Ethylenglykoldiglycidylether, Polyethylenglykol-diglycidylether, Glycerindiglycidylether, Glycerinpolyglycidylether, Diglycerinpolyglycidylether, Polyglycerinpolyglycidylether, Sorbitpolyglycidylether, Pentaerythritpolyglycidylether, Propylenglykoldiglycidylether und Polypropylenglykoldiglycidylether, Polyaziridinverbindungen wie 2,2-Bishydroxymethylbutanol-tris[3-(1-aziridinyl)-propionat], Diamide der Kohlensäure, wie 1,6-Hexamethylendiethylenharnstoff, Diphenylmethan-bis-4,4'-N,N'-diethylenharnstoff, Halogenepoxyverbindungen, wie Epichlorhydrin und $\alpha$-Methylepifluorhydrin, Polyisocyanate, wie 2,4-Toluylendiisocyanat und Hexamethylendiisocyanat, Alkylencarbonate wie 1,3-Dioxolan-2-on und 4-Methyl-1,3-dioxolan-2-on, weiterhin Bisoxazoline und Oxazolidone, Polyamidoamine sowie deren Umsetzungsprodukte mit Epichlorhydrin, ferner polyquaternäre Amine, wie Kondensationsprodukte von Dimethylamin mit Epichlorhydrin, Homo- und Copolymere von Diallyldimethylammoniumchlorid sowie Homo- und Copolymerisate von Dimethylaminoethyl(meth)acrylat, die gegebenenfalls mit beispielsweise Methylchlorid quaterniert sind.

Die wasserabsorbierenden Polymerisate können hergestellt werden, indem man die Monomere A, B und C, gegebenenfalls in Gegenwart einer geeigneten Pfropfgrundlage, in wässriger Lösung einer radikalischen Polymerisation unterwirft. Die Polymerisation kann sowohl in homogener wässriger Phase als auch als Suspensionspolymerisation erfolgen, wobei die wässrige Lösung der Monomere die disperse Phase bildet.

Bevorzugt ist die Polymerisation in wässriger Lösung als sogenannte Gelpolymerisation. Hierzu wird z. B. eine 10 bis 70 gew.-%ige wässrige Lösung der Monomere A, B und C, gegebenenfalls in Gegenwart einer geeigneten Pfropfgrundlage, mittels eines Polymerisationsinitiators unter Ausnutzung des Trommsdorff-Norrish-Effektes polymerisiert.

Die Polymerisation erfolgt in der Regel im Temperaturbereich von 0 °C und 150 °C, vorzugsweise im Bereich von 10 °C und 100 °C, und kann sowohl bei Normaldruck als auch unter erhöhtem oder erniedrigtem Druck durchgeführt werden. Wie üblich kann die Polymerisation auch in einer Schutzgasatmosphäre, vorzugsweise unter Stickstoff, ausgeführt werden.

Als technische Verfahren zur Herstellung dieser Produkte können alle Verfahren Anwendung finden, die üblicherweise bei der Herstellung von Superabsorbern eingesetzt werden. Geeignete Maßnahmen sind z. B. in "Modern Superabsorbent Polymer Technology", F.L. Buchholz and A.T. Graham, Wiley-VCH, 1998, Kapitel 3, erläutert, worauf

hiermit Bezug genommen wird. Als Polymerisationsreaktoren kommen die zur Herstellung üblichen Reaktoren, im Falle der Lösungspolymerisation insbesondere Bandreaktoren und Kneter, in Betracht (siehe "Modern Superabsorbent Polymer Technology", Kapitel 3.2.3). Die Polymerisate werden besonders bevorzugt nach einem kontinuierlichen oder diskontinuierlichen Knetverfahren hergestellt.

Als Initiatoren kommen grundsätzlich alle Verbindungen in Betracht, die beim Erwärmen auf Polymerisationstemperatur unter Bildung von Radikalen zerfallen. Die Polymerisation kann auch durch Einwirkung energiereicher Strahlung, z. B. UV-Strahlung, in Gegenwart von Photoinitiatoren ausgelöst werden. Auch eine Initiierung der Polymerisation durch Einwirkung von Elektronenstrahlen auf die polymerisierbare, wässrige Mischung ist möglich.

Geeignete Initiatoren sind beispielsweise Peroxoverbindungen wie organische Peroxide, organische Hydroperoxide, Wasserstoffperoxid, Persulfate, Perborate, Azoverbindungen und die sogenannten Redoxkatalysatoren. Bevorzugt werden wasserlösliche Initiatoren. In manchen Fällen ist es vorteilhaft, Mischungen verschiedener Polymerisationsinitiatoren zu verwenden, z. B. Mischungen aus Wasserstoffperoxid und Natrium- oder Kaliumperoxodisulfat. Geeignete organische Peroxide sind beispielsweise Acetylacetonperoxid, Methylethylketonperoxid, tert-Butylhydroperoxid, Cumolhydroperoxid, tert-Amylperpivalat, tert-Butylperpivalat, tert-Butylperneohexanoat, tert-Butylperisobutyrat, tert-Butyl-per-2-ethylhexanoat, tert-Butylperisononanoat, tert-Butylpermaleat, tert-Butylperbenzoat, Di-(2-ethylhexyl)peroxidicarbonat, Dicyclohexylperoxidicarbonat, Di-(4-tert.-butylcyclohexyl)peroxidicarbonat, Dimyristilperoxidicarbonat, Diacetylperoxi-dicarbonat, Allylperester, Cumylperoxyneodecanoat, tert.-Butyl-per-3,5,5-trimethylhexanoat, Acetylcyclohexylsulfonylperoxid, Dilaurylperoxid, Dibenzoylperoxid und tert.-Amylperneodekanoat. Besonders geeignete Polymerisationsinitiatoren sind wasserlösliche Azostarter, z. B. 2,2'-Azo-bis-(2-amidinopropan)dihydrochlorid, 2,2'-Azobis-(N,N'-dimethylen)isobutyramidin-dihydrochlorid, 2-(Carbamoylazo)isobutyronitril, 2,2'-Azobis[2-(2'-imidazolin-2-yl)propan]dihydrochlorid und 4,4'-Azobis-(4-cyanovaleriansäure). Die genannten Polymerisationsinitiatoren werden in üblichen Mengen eingesetzt, z. B. in Mengen von 0,01 bis 5, vorzugsweise 0,05 bis 2,0 Gew.-%, bezogen auf die zu polymerisierenden Monomeren.

Die bevorzugten Redoxinitiatoren zählen zu den wasserlöslichen Initiatoren und enthalten als oxidierende Komponente mindestens eine der oben angegebenen Peroxoverbindungen und als reduzierende Komponente beispielsweise Ascorbinsäure, Glukose, Sorbose, Ammonium- oder Alkalimetallhydrogensulfit, -sulfit, -thiosulfat, -hyposulfit, -pyro-sulfit oder -sulfid, Metallsalze, wie Eisen(II)-ionen oder Natriumhydroxymethylsulfoxylat. Vorzugsweise verwendet man als reduzierende Komponente des Redoxkatalysators Ascorbinsäure oder Natriumsulfit. Bezogen auf die bei der Polymerisation eingesetzte Menge an Monomeren verwendet man beispielsweise $3 \times 10^{-6}$ bis 1 Mol-% der reduzierenden Komponente des Redoxkatalysatorsystems und 0,001 bis 5,0 Mol-% der oxidierenden Komponente des Redoxkatalysators.

Wenn man die Polymerisation durch Einwirkung energiereicher Strahlung auslöst, verwendet man üblicherweise als Initiator sogenannte Photoinitiatoren.

Bei der nachträglichen Vernetzung werden Polymere, die durch die Polymerisation der oben genannten monoethylenisch ungesättigten Säuren und gegebenfalls monoethylenisch ungesättigten Comonomere hergestellt wurden und die typischerweise ein Molekulargewicht größer 5000, bevorzugt größer 50000 aufweisen, mit Verbindungen umgesetzt, die mindestens zwei gegenüber Säuregruppen reaktive Gruppen aufweisen. Diese Umsetzung kann bei Raumtemperatur oder aber bei erhöhten Temperaturen bis zu 220 °C erfolgen. Als Vernetzer fungieren die vorstehend erwähnten Monomere C, die wenigstens zwei funktionelle Gruppe mit komplementärer Reaktivität gegenüber Carboxylgruppen aufweisen.

Zur nachträglichen Vernetzung werden die Vernetzer den erhaltenen Polymeren in Mengen von 0,5 bis 20 Gew.-%, bevorzugt von 1 bis 14 Gew.-%, bezogen auf die Menge des Polymers, zugesetzt.

Die erfindungsgemäßen Polymerisate fallen nach der Polymerisation in der Regel als Hydrogele mit einem Feuchtigkeitsgehalt von z. B. 0 bis 90 Gew.-%, meist 20 bis 90 Gew.-% an, die in der Regel zunächst nach bekannten Methoden grob zerkleinert werden. Die Grobzerkleinerung der Hydrogele erfolgt mittels üblicher Reiß-und/oder Schneidwerkzeuge, z. B. durch die Wirkung einer Austragspumpe im Falle der Polymerisation in einem zylindrischen Reaktor oder durch eine Schneidwalze oder Schneidwalzenkombination im Falle der Bandpolymerisation.

Sofern die Monomere A in nicht neutralisierter Form eingesetzt worden sind, kann man das erhaltene saure Polymerisat auf den gewünschten Neutralisationsgrad von in der Regel wenigstens 25 bis 90 mol-%, vorzugsweise 50 bis 80 mol-%, insbesondere 65 bis 80 mol-% und ganz besonders bevorzugt 70 bis 78 mol.-%, bezogen auf Säuregruppen tragende Monomereinheiten, bringen. Alternativ kann die Einstellung des Neutralisationsgrades auch vor oder während der Polymerisation, z. B. im Kneter, vorgenommen werden.

Als Neutralisationsmittel kommen Alkalimetallbasen oder Ammoniak bzw. Amine in Frage. Vorzugsweise wird Natronlauge oder Kalilauge verwendet. Die Neutralisation kann jedoch auch mit Hilfe von Natriumcarbonat, Natriumhydrogencarbonat, Kaliumcarbonat oder Kaliumhydrogencarbonat oder anderen Carbonaten oder Hydrogencarbonaten oder Ammoniak vorgenommen werden. Darüberhinaus können für die Neutralisation primäre, sekundäre und tertiäre Amine eingesetzt werden.

Das wasserabsorbierende Polymerisat weist üblicherweise einen pH-Wert im Bereich von 4,0 bis 7,5, vorzugsweise im Bereich von 5,0 bis 7,5, insbesondere im Bereich von 5,5 bis 7,0 und ganz besonders bevorzugt im Bereich von 5,5 bis 6,5 auf.

Das so erhaltene, vorzugsweise (teil)neutralisierte Polymerisat wird anschließend bei erhöhter Temperatur, z. B. im Bereich von 80 °C bis 250 °C und insbesondere im Bereich von 100 °C bis 180 °C, nach bekannten Verfahren getrocknet (siehe "Modern Superabsorbent Polymer Technology" Kapitel 3.2.5). Hierbei erhält man die Polymerisate in Form von Pulvern oder Granulaten, die gegebenenfalls zur Einstellung der Partikelgröße noch mehreren Mahl- und Siebvorgängen unterworfen werden (siehe "Modern Superabsorbent Polymer Technology" Kapitel 3.2.6 und 3.2.7).

Vorzugsweise wird das erhaltene teilchenförmige Polymerisat anschließend oberflächennachvernetzt. Hierzu werden Verbindungen, die mit den sauren funktionellen Gruppen der Polymere unter Vernetzung reagieren können, vorzugsweise in Form einer wasserhaltigen Lösung auf die Oberfläche der Polymerisat-Partikel aufgebracht. Die wasserhaltige Lösung kann wassermischbare organische Lösungsmittel enthalten. Geeignete Lösungsmittel sind Alkohole wie Methanol, Ethanol, Isopropanol oder Aceton.

Geeignete Nachvernetzungsmittel sind beispielsweise:

- Di- oder Polyglycidylverbindungen wie Phosphonsäurediglycidylether oder Ethylenglykoldiglycidylether, Bischlorhydrinether von Polyalkylenglykolen,
- Alkoxysilylverbindungen,
- Polyaziridine, Aziridin-Einheiten enthaltende Verbindungen auf Basis von Polyethern oder substituierten Kohlenwasserstoffen, beispielsweise Bis-N-aziridinomethan,
- Polyamine oder Polyamidoamine sowie deren Umsetzungsprodukte mit Epichlorhydrin,
- Polyole wie Ethylenglykol, 1,2-Propandiol, 1,4-Butandiol, Glycerin, Methyltriglykol, Polyethylenglykole mit einem mittleren Molekulargewicht $M_w$ von 200 - 10000, Di- und Polyglycerin, Pentaerythrit, Sorbit, die Oxethylate dieser Polyole sowie deren Ester mit Carbonsäuren oder der Kohlensäure wie Ethylencarbonat oder Propylencarbonat,
- Kohlensäurederivate wie Harnstoff, Thioharnstoff, Guanidin, Dicyandiamid, 2-Oxazolidinon und dessen Derivate, Bisoxazolin, Polyoxazoline, Di- und Polyisocyanate,
- Di- und Poly-N-methylolverbindungen wie beispielsweise Methylenbis(N-methylol-methacrylamid) oder Melamin-FormaldehydHarze,
- Verbindungen mit zwei oder mehr blockierten Isocyanat-Gruppen wie beispielsweise Trimethylhexamethylendiisocyanat blockiert mit 2,2,3,6-Tetramethyl-piperidinon-4,
- Salze mehrwertiger Metalle, wie beispielsweise Aluminiumsulfat.

Bei Bedarf können saure Katalysatoren wie p-Toluolsulfonsäure, Phosphorsäure, Borsäure oder Ammoniumdihydrogenphosphat zugesetzt werden.

Besonders geeignete Nachvernetzungsmittel sind Di- oder Polyglycidylverbindungen wie Ethylenglykoldiglycidylether, die Umsetzungsprodukte von Polyamidoaminen mit Epichlorhydrin und 2-Oxazolidinon.

Das Aufbringen der Vernetzer-Lösung erfolgt bevorzugt durch Aufsprühen einer Lösung des Vernetzers in herkömmlichen Reaktionsmischern oder Misch- und Trocknungsanlagen wie beispielsweise Patterson-Kelly-Mischer, DRAIS-Turbulenzmischer, Lödige-Mischer, Schneckenmischer, Tellermischer, Wirbelschichtmischer und Schugi-Mix. Nach Aufsprühen der Vernetzer-Lösung kann ein Temperaturbehandlungsschritt nachfolgen, bevorzugt in einem nachgeschalteten Trockner, bei einer Temperatur zwischen 80 und 230 °C, bevorzugt 80 bis 190 °C, und besonders bevorzugt zwischen 100 und 160 °C, über einen Zeitraum von 5 Minuten bis 6 Stunden, bevorzugt 10 Minuten bis 2 Stunden und besonders bevorzugt 10 Minuten bis 1 Stunde, wobei sowohl Spaltprodukte als auch Lösungsmittelanteile entfernt werden können. Die Trocknung kann aber auch im Mischer selbst erfolgen, durch Beheizung des Mantels oder Einblasen eines vorgewärmten Trägergases. Gegebenenfalls enthält die Vernetzer-Lösung wenigstens ein Tensid.

Üblicherweise weist das wasserabsorbierende Polymerisat eine Partikelgöße von weniger als 850 μm, vorzugsweise 100 bis 600 μm, insbesondere 100 bis 500 μm auf.

Der Gehalt an Extrahierbaren beträgt nach 16 stündiger Extraktion (mit 0,9 Gew.-%iger wässriger NaCl-Lösung) üblicherweise weniger als 30 Gew.-%, vorzugsweise weniger als 22 Gew.-% und insbesondere weniger als 15 Gew.-%, bezogen auf das Gewicht des wasserabsorbierenden Polymerisats.

Das erfindungsgemäße wasserabsorbierende Mittel kann außerdem einen Träger enthalten. Gewöhnlich ist

der Träger ein Fasermaterial, das unter Cellulose, modifizierter Cellulose, Rayon, Polyester, Polyester wie Polyethylenterephthalat, Polypropylen, hydrophilisiertem Nylon, Polyethylen, Polyacryl, Polyamiden, Polystyrol, Polyurethan, Polyacrylnitril ausgewählt ist. Besonders bevorzugt werden Cellulosefasern wie Zellstoff verwendet. Die Fasern haben in der Regel einen Durchmesser von 1 bis 200 μm, bevorzugt 10 bis 100 μm. Darüberhinaus haben die Fasern vorzugsweise eine Mindestlänge von etwa 1 mm.

Der Anteil des Fasermaterials bezogen auf die Gesamtmenge des wasserabsorbierenden Polymerisats beträgt üblicherweise bis zu 10000 Gew.-%, bevorzugt 0 bis 100 Gew.-% und besonders bevorzugt weniger als 60 Gew.-%.

Zur Herstellung des erfindungsgemäßen wasserabsorbierenden Mittels geht man von einem teilchenförmigen wasserabsorbierenden Polymerisat aus, das in getrockneter Form oder als zerkleinertes Hydrogel vorliegt und bringt die Teilchen in Kontakt mit dem stickstoffhaltigen Polymer, z. B. indem man auf die Oberfläche der Teilchen des wasserabsorbierenden Polymerisates das stickstoffhaltige Polymer, vorzugsweise in Form einer Lösung aufbringt. In der Regel führt man abschließend einen Trocknungsschritt durch. Alternativ kann man die Teilchen des wasserabsorbierenden Polymerisates mit einem Träger in Kontakt bringen, auf den man zuvor das stickstoffhaltige Polymer aufgebracht hat.

Geeignete Lösungsmittel für das stickstoffhaltige Polymer sind Wasser oder organische Lösungsmittel, wie Alkohole, beispielsweise Methanol, Ethanol, Isopropanol, Ketone, beispielsweise Aceton, Methylethylketon oder Gemische von Wasser mit den vorstehend genannten organischen Lösungsmitteln. Soweit man als stickstoffhaltiges Polymer ein (teil)hydrolysiertes Polyvinylamid verwendet, kann die bei der (Partial)hydrolyse erhaltene Lösung unmittelbar verwendet werden. Die Konzentration des stickstoffhaltigen Polymers in dem Lösungsmittel kann innerhalb weiter Grenzen variiert werden. Im Allgemeinen liegt sie im Bereich von 0,1 bis 20 Gew.-%, vorzugsweise 1 bis 15 Gew.-%.

Gegebenenfalls kann die Lösung des stickstoffhaltigen Polymers ein Tensid enthalten. Alternativ kann man vor dem Aufbringen der Lösung des stickstoffhaltigen Polymers auf das wasserabsorbierende Polymerisat zunächst ein Tensid aufbringen. Das Aufbringen des Tensids kann beispielsweise während der Oberflächennachvernetzung erfolgen. Das Tensid dient dazu, die Oberflächenspannung der Lösung herabzusetzen und eine gleichmäßige Benetzung zu fördern. Geeignete Tenside sind nichtionische, anionische und kationische Tenside sowie Gemische davon. Vorzugsweise enthält das wasserabsorbierende Mittel nichtionische Tenside. Als nichtionische Tenside eignen sich beispielsweise Sorbitanester, wie die Mono-, Di- oder Triester der Sorbitane mit $C_8$-$C_{18}$-Carbonsäuren wie Laurin-, Palmitin-, Stearin- und Ölsäure; Polysorbate; Alkylpolyglucoside mit 8 bis 22, vorzugsweise 10 bis 18 Kohlenstoffatomen in der Alkylkette und 1 bis 20, vorzugsweise 1,1 bis 5 Glucosideinheiten; N-Alkylglucamide; Alkylaminalkoxilate oder Alkylamidethoxylate; alkoxylierte $C_8$-$C_{22}$-Alkohole wie Fettalkoholalkoxylate oder Oxoalkoholalkoxylate; Blockpolymerisate von Ethylenoxid, Propylenoxid und/oder Butylenoxid; Alkylphenolethoxylate mit $C_6$-$C_{14}$-Alkylketten und 5 bis 30 Mol Ethylenoxideinheiten.

Die Menge des Tensids beträgt in der Regel 0,01 bis 0,5 Gew.-%, bevorzugt weniger als 0,1 Gew.-%, und liegt insbesondere unterhalb 0,05 Gew.-% bezogen auf das Gewicht des wasserabsorbierenden Polymerisat.

In einer bevorzugten Ausführungsform sprüht man die Lösung des stickstoffhaltiges Polymers in einem Reaktionsmischer oder einer Misch- und Trocknungsanlage, wie beispielsweise einem Patterson-Kelly-Mischer, DRAIS-Turbulenzmischer, Lödige-Mischer, Schneckenmischer, Tellermischer, Wirbelschichtmischer oder Schugi-Mix, auf das wasserabsorbierende Polymerisat auf. Das Auftragen erfolgt in der Regel bei Temperaturen zwischen Raumtemperatur und 100 °C. Üblicherweise erfolgt das Auftragen im Anschluss an die Trocknung der (oberflächen)nachvernetzten, häufig noch warmen Teilchen des wasserabsorbierenden Polymerisates.

Nach Aufsprühen der Lösung erfolgt üblicherweise ein Temperaturbehandlungsschritt, bevorzugt in einem nachgeschalteten Trockner, bei einer Temperatur zwischen 40 und 140 °C, bevorzugt 40 bis 120 °C, und besonders bevorzugt zwischen 60 und 120 °C. Üblicherweise führt man den Temperaturnachbehandlungsschritt solange aus, bis das wasserabsorbierende Mittel den gewünschten Tocknungsgrad aufweist. Der Restfeuchtigkeitsgehalt liegt in der Regel unterhalb 5 Gew.-%, vorzugsweise unterhalb 3 Gew.-%, besonders bevorzugt unterhalb 2 Gew.-% und ganz besonders bevorzugt zwischen 0,01 bis 1 Gew.-%, bezogen auf das Gewicht des wasserabsorbierenden Mittels. Die Trocknung kann aber auch im Mischer selbst erfolgen, durch Beheizung des Mantels oder Einblasen eines vorgewärmten Trägergases. Der Temperaturnachbehandlungsschritt verbessert die Rieselfähigkeit des wasserabsorbierenden Mittels.

Bei Trocknungstemperaturen von mehr als 140 °C nimmt die SFC des erhaltenen wasserabsorbierenden Mittels zwar zu, aber die Nassfestigkeit des wasserabsorbierenden Mittels verschlechtert sich. Vermutlich bilden sich bei hohen Temperaturen übermäßig viele kovalente Bindungen zwischen dem stickstoffhaltigen Polymer und z.B. den Carboxylgruppen des wasserabsorbierenden Polymerisats aus. Dies führt zur Herabsetzung der Beweglichkeit des stickstoffhaltigen Polymers und die Fähigkeit des stickstoffhaltigen Polymers nimmt ab, ein Teilchen des wasserabsorbierenden Polymerisats mit einem anderen zu verknüpfen.

Alternativ kann man auch den zuvor genannten Träger mit dem stickstoffhaltigen Polymer beschichten, z. B. durch Aufsprühen einer Lösung des stickstoffhaltigen Polymers. Anschließend mischt man den beschichteten Träger gründlich mit den Teilchen des wasserabsorbierenden Polymerisats oder platziert den beschichteten Träger in unmittelbarer Nachbarschaft zum wasserabsorbierenden Polymerisat.

Sofern man ein feinteiliges, wasserunlösliches Salz und gegebenenfalls ein Staubbindemittel mitverwendet, kann der Auftrag oder das Vermischen mit diesen Bestandteilen vor, gleichzeitig oder nach dem Auftrag des stickstoffhaltigen Polymers erfolgen.

Das Aufbringen des feinteiligen, wasserunlöslichen Salzes kann beispielsweise durch inniges Vermischen erfolgen. Üblicherweise fügt man das feinteilige, wasserunlösliche Salz bei Raumtemperatur zum teilchenförmigen, wasserabsorbierenden Polymerisat und mischt solange, bis eine homogene Mischung vorliegt. Das Mischen kann unter Verwendung üblicher Vorrichtungen, beispielsweise mit einem Trommelmischer, Bandschneckenmischer oder Siloschneckenmischer, erfolgen. Das Vermischen mit dem feinteiligen wasserunlöslichen Salz kann vor oder nach einer Oberflächennachvernetzung erfolgen, z. B. während des Temperaturnachbehandlungsschrittes nach dem Aufbringen des Nachvernetzungsmittels.

Das Staubbindemittel wird zweckmäßigerweise in Form einer wässrigen Lösung aufgebracht, vorzugsweise während oder nach dem innigen Mischen mit dem wasserunlöslichen Salz. Man kann das Staubbindemittel auch in die Lösung des stickstoffhaltigen Polymers mit einbeziehen.

Das erfindungsgemäße wasserabsorbierende Mittel eignet sich hervorragend als Absorptionsmittel für Wasser und wässrige Flüssigkeiten, insbesondere Körperflüssigkeiten. Es kann mit Vorteil zur Herstellung von Hygieneartikeln wie Windeln, Inkontinenzeinlagen und -hosen, Tampons oder Damenbinden verwendet werden. Es eignet sich ferner zur Absorption von Wundflüssigkeit in Pflastern, Kompressen und sonstigen Wundauflagen. Sie können ferner zur Bodenverbesserung, z. B. als Wasser zurückhaltendes Mittel im landwirtschaftlichen Gartenbau, verwendet werden.

Die folgenden Beispiele sollen die Erfindung näher erläutern, ohne sie jedoch einzuschränken.

I Beschreibung der Testmethoden

1. Zentrifugenretentionskapazität (CRC: Centrifuge Retention Capacity)
Hierbei wird die freie Quellbarkeit des Hydrogel-bildenden Polymerisats im Teebeutel bestimmt. Zur Bestimmung der CRC werden 0,2000 ± 0,0050 g getrocknetes Polymerisat in einem 60 x 85 mm großen Teebeutel eingewogen, der anschließend verschweißt wird. Der Teebeutel wird für 30 Minuten in 0,9 gew.%-ige Kochsalzlösung gegeben (mindestens 0,83 1 KochsalzLösung/1 g Polymerpulver). Anschließend wird der Teebeutel 3 Minuten lang bei 250 G zentrifugiert und danach zur Bestimmung der absorbierten Flüssigkeitsmenge gewogen.

2. Absorption unter Druck (AUL Absorbency Under Load) (0,7 psi)
Die Meßzelle zur Bestimmung der AUL 0,7 psi ist ein Plexiglas-Zylinder mit einem Innendurchmesser von 60 mm und einer Höhe von 50 mm, der an der Unterseite einen angeklebten Edelstahl-Siebboden mit einer Maschenweite von 36 μm aufweist. Zu der Meßzelle gehört weiterhin eine Plastikplatte mit einem Durchmesser von 59 mm und ein Gewicht, welches zusammen mit der Plastikplatte in die Meßzelle hineingestellt werden kann. Das Gewicht der Plastikplatte und das Gewicht betragen zusammen 1345 g. Zur Durchführung der Bestimmung der AUL 0,7 psi wird das Gewicht des leeren Plexiglas-Zylinders und der Plastikplatte ermittelt und als $W_0$ notiert. Dann werden 0,900 ± 0,005 g wasserabsorbierndes Polymerisat in den Plexiglas-Zylinder eingewogen und möglichst gleichmäßig auf dem Edelstahl-Siebboden verteilt. Anschließend wird die Plastikplatte vorsichtig in den Plexiglas-Zylinder hineingelegt und die gesamte Einheit gewogen; das Gewicht wird als Wa notiert. Nun wird das Gewicht auf die Plastikplatte in dem Plexiglas-Zylinder gestellt. In die Mitte der Petrischale mit einem Durchmesser von 200 mm und einer Höhe von 30 mm wird eine keramische Filterplatte mit einem Durchmesser von 120 mm und einer Porosität 0 gelegt und soviel 0,9 gew.-%ige Natriumchloridlösung eingefüllt, dass die Flüssigkeitsoberfläche mit der Filterplattenoberfläche abschließt, ohne dass die Oberfläche der Filterplatte bedeckt wird. Anschließend wird ein rundes Filterpapier mit einem Durchmesser von 90 mm und einer Porengröße < 20 μm (S&S 589 Schwarzband von Schleicher & Schüll) auf die keramische Platte gelegt. Der Plexiglas-Zylinder mit dem wasserabsorbiernden Polymerisat wird mit der Plastikplatte und dem Gewicht nun auf das Filterpapier gestellt und dort für 60 Minuten belassen. Nach dieser Zeit wird die komplette Einheit aus der Petrischale vom Filterpapier herausgenommen und anschließend das Gewicht aus dem Plexiglas-Zylinder entfernt. Der das gequollene Polymerisat enthaltende Plexiglas-Zylinder wird zusammen mit der Plastikplatte ausgewogen und das Gewicht als $W_b$ notiert.
Die Absorption unter Druck (AUL) wird wie folgt berechnet:

$$\text{AUL 0,7 psi [g/g]} = [W_b - W_a] / [W_a - W_0]$$

3. Saline Flow Conductivity (SFC)

Die Testmethode zur Bestimmung der SFC ist in der US 5,599,335 beschrieben.

4. Ball Burst Strength (BBS)
Die Testmethode zur Bestimmung der BBS (30 min), d. h. vom Beginn der Quellung des Superabsorbers bis zur Messung vergehen 30 Minuten, ist in der US 6,121,509 beschrieben. Die Bestimmung der BBS (16 h) erfolgt analog zu der Bestimmung der BBS (30 min), aber vom Beginn der Quellung des Superabsorbers bis zur Messung vergehen 16 Stunden. In Abänderung zur US 6,121,509 steht das Vorratsgefäß auf der Waage und die Meszellen auf höhenverstellbaren Plattformen. Nach 30 Minuten bzw. 16 Stunden entfernt man das Stahlgewicht und überführt die Probe zur Messung in die Messapparatur. Die BBS ist ein Maß für die Nassfestigkeit des wasserabsorbierenden Polymerisates.
Die relative Abnahme der BBS (16 h vs. 30 min) wird wie folgt berechnet:

$$\text{BBS-Abnahme} = \frac{\text{BBS (30 min)} - \text{BBS (16 h)}}{\text{BBS (30 min)}} \times 100\ \%$$

5. Extrahierbarer Anteil (16 h)
Die Bestimmung des extrahierbaren Anteils nach 16 Stunden erfolgt gemäß ISO/DIS 17190-10 (erhältlich über EDANA (European Disposables and Nonwovens Association)).

II Herstellungsbeispiele

Beispiele

Herstellung eines oberflächennachvernetzten Polymerisats

Man stellte nach einem üblichen Verfahren ein Grundpolymerisat mit einer Zentrifugenretentionskapazität von 30 - 31 g/g her, das aus Acrylsäure, Natriumacrylat und ethoxyliertem Trimethylolpropantriacrylat aufgebaut ist und einen Neutralisationsgrad der Acrylsäure von 75 Mol-% aufweist. Das nach Beendigung der Polymerisation erhaltene Gel wurde mechanisch zerkleinert. Das zerkleinerte Gel wurde dann im Labortrockenschrank getrocknet, mit einem Laborwalzenstuhl gemahlen und schließlich bei 150 bis 850 µm abgesiebt. Das erhaltene Grundpolymer wurde in einem Pulvermischaggregat mittels einer Zweistoffdüse mit einer wässrigen Oberflächennachvernetzer-Lösung (0,08 Gew.-% Oxazolidinon, 0,02 Gew.-Sorbitanmonolaurat und 3,5 Gew.-% 1,2-Propandiol, jeweils bezogen auf das Basispolymer) besprüht, danach sprühte man 0,5 Gew.-% Aluminiumsulfat (als wässrige 26,8 %ige Lösung), bezogen auf das Grundpolymerisat auf, und temperte von etwa 80 min bei einer Temperatur von 175 - 180 °C. Man ließ auf Raumtemperatur abkühlen und siebte auf eine Korngröße von 150 - 850 µm ab, um Klumpen zu entfernen. Das Polymerisat besaß folgende Korngrößenverteilung: 0,14 % (größer 850 µm), 35,8 % (300 - 600 µm), 63,3 % (150 - 300 µm) und weniger als 0,1 % (kleiner 150 µm). Die Kennzahlen des Polymerisats sind zum Vergleich in Tabelle 1 angegeben.
1200 g des vorstehend hergestellten Polymerisats wurden in einem 5 1 Lödige-Pflugschar-Labormischer bei Raumtemperatur vorgelegt und danach 13 Minuten unter Mischen bei 200 U/min mittels einer Zweistoffdüse (Stickstoff als Zerstäubungsgas bei einem Druck von etwa 1 bar, die Flüssigkeit wurde über eine Pumpe eingespeist) mit 65,71 g einer 7,3 Gew.-%igen wässrigen Polyvinylamin-Lösung unterschiedlichen Hydrolysegrades (Bsp. 1: Basocoll PR 8086, Hydrolysegrad 95 mol %; Beispiel 2: Basocoll Pr 8092, Hydrolysegrad 75 mol %; Bsp. 3: Luredur PR 8097, Hydrolysegrad 44 mol %; Bsp. 4: Basocoll PR 8095, Hydrolysegrad 31 mol %; Bsp. 5: Basocoll PR 8094, Hydrolysegrad 14 mol %, jeweils mit einem gewichtsmittleren Molekulargewicht von etwa 400 000 Dalton) besprüht. Das erhaltene Produkt wurde anschließend in einen analogen vorgeheizten Lödige-Pflugschar-Labormischer überführt und bei einer Umdrehungsgeschwindigkeit von 50 U/min etwa 60 min bei 100 °C getrocknet. Der Anteil des stickstoffhaltigen Polymers betrug jeweils 0,4 Gew.-% bezogen auf das absorbierende Polymerisat. Die Kennzahlen der erhaltenen Produkte sind in der nachfolgenden Tabelle 1 angegeben.
Wie man der Tabelle 1 entnehmen kann, nimmt die SFC und die BBS in den Beispielen 2 und 3 im Vergleich zu den nicht erfindungsgemäßen Beispielen deutlich zu.
Des Weiteren wurde die Lagerstabilität des wasserabsorbierenden Mittels untersucht. Die Ergebnisse sind in der nachfolgenden Tabelle 2 angegeben. Die wasserabsorbierenden Mittel in den Beispielen 6 bis 10 wurden auf die zuvor beschriebene Weise hergestellt (Bsp. 6 und 7: Basocoll PR 8144, welches durch Ultrafiltration von den Nebenprodukten der Hydrolyse gereinigt wurde, Hydrolysegrad 95 mol %; Bsp. 8, 9 und 10: Luredur PR 8097, Hydrolysegrad 44 mol %). Der Anteil des stickstoffhaltigen Polymers betrug jeweils 0,4 Gew.-%, bezogen auf das absorbierende Polymerisat. Das wasserabsorbierende Mittel in Beispiel 10 wurde jedoch auf eine Korngröße von 150 bis 500 µm

abgesiebt. Die Messungen erfolgten in den Beispielen 6, 8 und 10 direkt nach der Herstellung und in den Beispielen 7 und 9 nach einer 14tägigen Lagerung bei 60 °C.

Die Ergebnisse aus Tabelle 2 belegen, dass nach trockener Lagerung bei 60 °C über 14 Tage die Abnahme an BBS und AUL in dem erfindungsgemäßen Beispiel 9 deutlich geringer ist als in dem Vergleichsbeispiel 7. Die Ergebnisse zeigen weiterhin, dass die Korngrößenverteilung des Polymerisats einen Einfluss auf SFC und BBS hat.

Tabelle 1:

| Beispiel | HG* [mol %] | protonierbare N-atome [mol/kg] | SFC x $10^{-7}$ [$cm^3 \cdot s/g$] | BBS (30 min) [gf] | BBS (16 h) [gf] | BBS-Abnahme (30 min → 16 h) [%] | CRC [g/g] | AUL 0,7 psi [g/g] |
|---|---|---|---|---|---|---|---|---|
| Vgl. | -- | -- | 111 | <10 | <10 | - | 27,5 | 23,4 |
| 1** | 95 | 21,4 | 133 | 169 | 160 | 5 | 27,1 | 19,0 |
| 2 | 75 | 15,0 | 138 | 196 | 150 | 23 | 26,9 | 19,2 |
| 3 | 44 | 7,5 | 229 | 169 | 110 | 35 | 27,3 | 20,6 |
| 4 | 31 | 5,0 | 104 | 55 | 27 | 51 | 26,9 | 21,9 |
| 5** | 14 | 2,1 | 111 | 21 | - | - | 26,9 | 21,1 |

HG*: Hydrolysegrad
** Vergleichsbeispiel

Tabelle 2:

| Beispiel | gewichtsmittleres Molekulargewicht des Polyvinyl amins [Dalton] | HG* [mol %] | protonierbare N-atome [mol/kg] | Lagerzustand | SFC x $10^{-7}$ [$cm^3 \cdot s/g$] | BBS (30 min) [gf] | CRC [g/g] | AUL 0,7 psi [g/g] | Korngrößenverteilung des Polymerisats [μm] |
|---|---|---|---|---|---|---|---|---|---|
| 6** | 35 000 | 95 | 21,4 | frisch her gestellt | 150 | 137 | 26,9 | 20,2 | 150 - 850 |
| 7** | 35 000 | 95 | 21,4 | 14 Tage bei 60 °C trocken gelagert | 255 | 73 | 26,8 | 19,1 | 150 - 850 |
| 8 | 400 000 | 44 | 7,5 | frisch her gestellt | 229 | 169 | 27,3 | 20,6 | 150 - 850 |
| 9 | 400 000 | 44 | 7,5 | 14 Tage bei 60 °C trocken gelagert | 200 | 160 | 26,9 | 20,5 | 150 - 850 |
| 10 | 400 000 | 44 | 7,5 | frisch her gestellt | 154 | 184 | 26,7 | 20,3 | 150 - 500 |

HG*: Hydrolysegrad

** Vergleichsbeispiel

## Patentansprüche

1. Wasserabsorbierendes Mittel, umfassend

   (a) Teilchen eines wasserabsorbierenden Polymerisats, und
   (b) ein stickstoffhaltiges Polymer, das 5 bis 17 mol/kg, bezogen auf das Gesamtgewicht des stickstoffhaltigen Polymers, protonierbare Stickstoffatome enthält.

2. Wasserabsorbierendes Mittel nach Anspruch 1, wobei das stickstoffhaltige Polymer ein Hydrolyseprodukt eines Homo- oder Copolymers eines N-Vinylcarbonsäureamids und/oder N-Vinylcarbonsäureimids ist.

3. Wasserabsorbierendes Mittel nach Anspruch 1 oder 2 mit einer

   - Korngrößenverteilung, worin mehr als 98 Gew.-% der Teilchen eine Korngröße von 100 bis 850 μm aufweisen,
   - Saline Flow Conductivity von wenigstens 30 x $10^{-7}$ $cm^3$ ·s/g,
   - Ball Burst Strength (30 min) von wenigstens 50 gf,
   - Ball Burst Strength (16 h) von wenigstens 50 gf und
   - einem Quotienten [BBS (30 min) - BBS (16 h)]/BBS (30 min) von weniger als 0,8.

4. Wasserabsorbierendes Mittel nach Anspruch 2, wobei das stickstoffhaltige Polymer ein Hydrolyseprodukt eines Homopolymerisat des N-Vinylformamids mit einem Hydrolysegrad von 30 bis 80 mol-% ist.

5. Wasserabsorbierendes Mittel nach Anspruch 1 oder 2, wobei das stickstoffhaltige Polymer ein gewichtsmittleres Molekulargewicht von 10000 bis 500000 Dalton aufweist.

6. Wasserabsorbierendes Mittel nach einem der vorhergehenden Ansprüche, umfassend 0,001 bis 5 Gew.-% stickstoffhaltiges Polymer, bezogen auf das Gewicht des wasserabsorbierenden Polymerisats.

7. Wasserabsorbierendes Mittel nach einem der vorhergehenden Ansprüche, zusätzlich umfassend ein feinteiliges, wasserunlösliches Salz.

8. Wasserabsorbierendes Mittel nach einem der vorhergehenden Ansprüche, wobei das wasserabsorbierende Polymerisat aufgebaut ist aus

   - 49,9 bis 99,9 Gew.-% wenigstens eines unter monoethylenisch ungesättigten Säuren und deren Salzen ausgewählten Monomers A,
   - 0 bis 50 Gew.-% wenigstens eines von dem Monomeren A verschiedenen monoethylenisch ungesättigten Monomers B und
   - 0,001 bis 20 Gew.-% wenigstens eines vernetzend wirkenden Monomers C.

9. Wasserabsorbierendes Mittel nach einem der vorhergehenden Ansprüche, wobei die Teilchen des wasserabsorbierenden Polymerisats oberflächennachvernetzt sind.

10. Wasserabsorbierendes Mittel nach einem der vorhergehenden Ansprüche, außerdem enthaltend einen Träger, der ausgewählt ist unter Zellulose, modifizierter Zellulose, Rayon, Polypropylen, Polyester, hydrophilisiertem Nylon, Polyethylen, Polyacryl, Polyamiden, Polystyrol, Polyurethan, Polyacrylnitril.

11. Wasserabsorbierendes Mittel nach Anspruch 10, wobei das stickstoffhaltige Polymer auf den Träger aufgebracht ist.

12. Verfahren zur Herstellung eines wasserabsorbierenden Mittels nach einem der vorhergehenden Ansprüche, wobei man das stickstoffhaltige Polymer oder eine Lösung davon auf die Teilchen des wasserabsorbierendes Polymerisat aufträgt und gegebenenfalls trocknet.

## Claims

1. A water absorbent comprising

(a) particles of a water absorbent polymer, and
(b) a nitrogenous polymer comprising from 5 to 17 mol/kg, based on the total weight of the nitrogenous polymer, of protonatable nitrogen atoms.

**2.** The water absorbent according to claim 1 wherein the nitrogenous polymer is a hydrolysis product of a homo- or copolymer of an N-vinylcarboxamide and/or N-vinylcarboximide.

**3.** The water absorbent according to claim 1 or 2 **characterized by**

- a particle size distribution wherein more than 98% by weight of the particles are from 100 to 850 μm in size,
- a Saline Flow Conductivity of at least 30 x $10^{-7}$ $cm^{3}\cdot$ s/g,
- a Ball Burst Strength (30 min) of at least 50 gf,
- a Ball Burst Strength (16 h) of at least 50 gf, and
- a quotient [BBS (30 min) - BBS (16 h)]/BBS (30 min) of less than 0.8.

**4.** The water absorbent according to claim 2 wherein the nitrogenous polymer is a hydrolysis product of a homopolymer of N-vinylformamide but has a degree of hydrolysis in the range from 30 to 80 mol%.

**5.** The water absorbent according to claim 1 or 2 wherein the nitrogenous polymer has a weight average molecular weight in the range from 10 000 to 500 000 daltons.

**6.** The water absorbent according to any preceding claim, comprising from 0.001% to 5% by weight of nitrogenous polymer, based on the weight of the water absorbent polymer.

**7.** The water absorbent according to any preceding claim, further comprising a finely divided water-insoluble salt.

**8.** The water absorbent according to any preceding claim wherein the water absorbent polymer is polymerized from

- from 49.9% to 99.9% by weight of at least one monomer A selected from the group consisting of monoethylenically unsaturated acids and salts thereof,
- from 0% to 50% by weight of at least one monoethylenically unsaturated monomer B other than said monomer A, and
- from 0.001% to 20% by weight of at least one crosslinking monomer C.

**9.** The water absorbent according to any preceding claim wherein the particles of the water absorbent polymer are surface postcrosslinked.

**10.** The water absorbent according to any preceding claim, further comprising a carrier selected from the group consisting of cellulose, modified cellulose, rayon, polypropylene, polyester, hydrophilicized nylon, polyethylene, polyacrylic, polyamides, polystyrene, polyurethane and polyacrylonitrile.

**11.** The water absorbent according to claim 10 wherein the nitrogenous polymer is applied onto the carrier.

**12.** A process for producing a water absorbent according to any preceding claims, which comprises the nitrogenous polymer or a solution thereof being applied onto the particles of the water absorbent polymer and, if appropriate, dried.

**Revendications**

**1.** Produit d'absorption d'eau, comprenant

(a) des particules d'un polymère absorbant l'eau, et
(b) un polymère azoté qui contient 5 à 17 moles/kg, par rapport au poids total du polymère azoté, d'atomes d'azote protonables.

**2.** Produit d'absorption d'eau suivant la revendication 1, dans lequel le polymère azoté est un produit d'hydrolyse d'un homopolymère ou copolymère d'un amide d'acide N-vinylcarboxylique et/ou d'un imide d'acide N-vinylcarboxylique.

**3.** Produit d'absorption d'eau suivant la revendication 1 ou 2, présentant

- une répartition granulométrique dans laquelle plus de 98 % en poids des particules présentent une taille de grain de 100 à 850 μm,
- une Saline Flow Conductivity d'au moins 30 x $10^{-7}$ $cm^3$. s/g,
- une Ball Burst Strength (30 minutes) d'au moins 50 gf,
- une Ball Burst Strength (16 heures) d'au moins 50 gf, et
- un quotient [BBS (30 min) - BBS (16 h)]/BBS (30 min) de moins de 0,8.

**4.** Produit d'absorption d'eau suivant la revendication 2, dans lequel le polymère azoté est un produit d'hydrolyse d'un homopolymère du N-vinylformamide ayant un degré d'hydrolyse de 30 à 80 % molaires.

**5.** Produit d'absorption d'eau suivant la revendication 1 ou 2, dans lequel le polymère azoté présente un poids moléculaire moyen pondéral de 10 000 à 500 000 daltons.

**6.** Produit d'absorption d'eau suivant l'une des revendications précédentes, comprenant 0,001 à 5 % en poids de polymère azoté, par rapport au poids du polymère absorbant l'eau.

**7.** Produit d'absorption d'eau suivant l'une des revendications précédentes, comportant en supplément un sel insoluble dans l'eau, finement divisé.

**8.** Produit d'absorption d'eau suivant l'une des revendications précédentes, dans lequel le polymère absorbant l'eau est constitué

- de 49,9 à 99,9 % en poids d'au moins un monomère A choisi parmi des acides monoéthyléniquement insaturés et leurs sels,
- de 0 à 50 % en poids d'au moins un monomère B monoéthyléniquement insaturé, différent du monomère A, et
- de 0,001 à 20 % en poids d'au moins un monomère C à action de réticulation.

**9.** Produit d'absorption d'eau suivant l'une des revendications précédentes, dans lequel les particules du polymère absorbant l'eau sont postréticulées en surface.

**10.** Produit d'absorption d'eau suivant l'une des revendications précédentes, contenant en outre un support qui est choisi parmi de la cellulose, de la cellulose modifiée, de la rayonne, du polypropylène, du polyester, du nylon hydrophilisé, du polyéthylène, du polyacryle, des polyamides, du polystyrène, du polyuréthanne, du polyacrylonitrile.

**11.** Produit d'absorption d'eau suivant la revendication 10, dans lequel le polymère azoté est appliqué sur le support.

**12.** Procédé de préparation d'un produit d'absorption d'eau suivant l'une des revendications précédentes, dans lequel on applique le polymère azoté ou une solution de celui-ci sur les particules du polymère absorbant l'eau et on sèche éventuellement.